# FASCICULE DE BREVET EUROPEEN

(11) **EP 0 905 125 B1**
(45) Date de publication et mention de la délivrance du brevet: **11.12.2002**
(21) Numéro de dépôt: 98402298.8
(22) Date de dépôt: 17.09.1998
(51) Int. Cl.: C07C 233/20, B01F 17/00

(54) **Composition utilisable comme agent de surface émulsifiant et dispersant et son procédé de fabrication**
Emulgierende und dispergierende Tenside und Verfahren zu deren Herstellung
Composition useful as emulsifying and dispersing surfactant and its preparation

(30) Priorité: 25.09.1997 FR 9712049
(43) Date de publication de la demande: 31.03.1999
(73) Titulaire: INSTITUT FRANCAIS DU PETROLE, 92502 Rueil-Malmaison (FR)
(72) Inventeur: Hillion, Gérard, 95220 Herblay (FR); Durand, Isabelle, 92500 Rueil Malmaison (FR); Stern, Robert, 75017 Paris (FR); Velly, Marie, 78360 Montesson (FR)

(56) Documents cités:
- BE-A- 543 960
- GB-A- 598 140
- US-A- 1 930 845
- US-A- 3 395 162
- US-A- 4 108 779

## Description

L'invention a pour objet une composition consistant en un mélange comprenant au moins des amides, des amines, des ester-amides, des ester-amines, des sels d'amines et des monoglycérides, tous dérivant d'acides gras monomères, dimères, trimères et/ou tétramères et un procédé pour sa fabrication, qui comprend la réaction de transamidification d'une huile polyinsaturée polymérisée, avec un aminoalcool.

L'invention concerne également les utilisations de cette composition, notamment comme agent émulsifiant permettant, selon la nature de l'huile, de former une émulsion huile-dans-eau ou eau-dans-huile, comme agent dispersant de solides ou comme stabilisant de mousses dans un liquide ou une émulsion.

Le mélange obtenu peut être utilisé, sans aucune purification, comme agent de surface émulsifiant et dispersant, à l'état pur, ou après dilution avec divers solvants comme, par exemple, des coupes aromatiques, divers alcools ou encore avec certains esters d'acides gras.

Il est connu que les produits issus de l'amidification d'un mélange d'oligomères d'acides gras, avec par exemple de la diéthanolamine, possèdent des propriétés émulsifiantes et dispersantes intéressantes dans diverses applications pour former des émulsions eau dans huile, ou huile dans eau, selon la nature de l'huile utilisée et des proportions respectives de ces deux constituants.

Par ailleurs, les articles de D. N. Bhattacharyya et al (J. Surface Sci. Technol., Vol. 5, No. 2, pp. 187-189, 1989, et Tenside Surf. Det. 27, 5, pp. 307-311, 1990), décrivent la préparation d'éthanolamides par réaction de diéthanolamine sur des "bodied oils" après saponification ou transestérification sous forme d'esters méthyliques d'huiles polymérisées thermiquement.

On a maintenant trouvé de façon surprenante qu'il était possible d'obtenir, d'une manière simple et économique, un produit possédant les mêmes propriétés tensioactives que les alcanolamides de dimères classiques en utilisant une autre source d'oligomères d'acides gras, qui est l'utilisation directe d'huiles polymérisées thermiquement.

L'objet de l'invention réside dans le fait que l'on obtient, en une seule étape, un produit, très bon marché, répondant à certaines caractéristiques tensioactives, dont un caractère amphiphile marqué, permettant de réaliser des émulsions eau-dans-huile ou huile-dans-eau.

L'élimination de quelques étapes importantes telles que :
- la dimérisation classique, avec des terres activées, des résines échangeuses d'ions ou des acides sulfoniques, suivie de l'élimination de ces catalyseurs ;
- l'amidification des oligomères d'acides gras avec l'élimination de l'eau de réaction ; et enfin
- l'élimination par filtration d'un sous-produit de réaction insoluble venant de la réaction de déshydratation de deux molécules de diéthanolamine (quand cet aminoalcool est utilisé), qui donne la N,N-bis(2-hydroxyéthyl) pipérazine, permet de réduire le temps de synthèse d'un facteur 5 à 6.

En effet, on obtient généralement en utilisant la voie de synthèse classique, un temps moyen de réaction de l'ordre d'une quinzaine d'heures, alors qu'en utilisant le mode opératoire de l'invention, un temps total moyen de trois heures est suffisant.

Il est connu que les huiles polyinsaturées, riches plus particulièrement en acides gras diéniques et triéniques peuvent, par action de la chaleur, se polymériser pour former des structures polymériques appelées « stand oil », « standolie », « bodied oil » ou, dans le cas spécifique de l'huile de lin, huile de lin épaissie par chauffage.

Cette polymérisation thermique ne peut être réalisée qu'en mettant en contact des chaînes grasses diéniques ou trièniques entre elles, comme par exemple l'acide linoléique et linolénique.

Cette réaction est obtenue par simple chauffage thermique à 300°C sous azote, ou à 280-290 °C sous pression réduite en présence d'anthraquinone ou de benzoquinone (voir Nisshin Oils Mills, Yokohama, Japan, Nagakura and Coll. (1975), 48 (4), 217-22) ou également catalysée par des métaux comme par exemple le zinc, le plomb, l'étain ou le cuivre, sous la forme de nitrates, de chlorures, de stéarates.

On peut citer comme exemple : le chauffage pendant 6 heures d'huile de lin avec de 0,5 à 2 % de nitrate de cuivre II [voir Sil S. and Koley S. N., Department of Chemical Technology, Univ. of Calcutta (1987) 37(8), 15-22].

Le temps de chauffage conditionne un gradient de viscosité à l'huile de lin polymérisée, qui peut atteindre, après un temps de chauffage supérieur à 20 heures, une viscosité de 65 Pa.s à 20 °C.

Mise à part l'huile de lin, on peut obtenir des huiles polymérisées en utilisant de l'huile de tournesol, de carthame, de bois de chine, de pépin de raisin, de soja, de maïs, ainsi que toutes les huiles possédant des teneurs élevées en acide linoléique et/ou linolénique.

On donne ci-après, à titre d'exemple, la composition des oligomères d'acides gras contenus dans une huile de lin polymérisée possédant une viscosité de 65 Pa.s à 20 °C :
- acides gras monomères = 44,10 %,
- acides gras dimères = 32,30 %,
- acides gras trimères = 14,50 % et
- oligomères supérieurs = 9,10 %.

Ces valeurs ont été obtenues après méthanolyse du triglycéride et leur séparation a été réalisée sur une chromatographie par perméation sur gel (GPC). L'indice d'acide de l'huile polymérisée est égal à 16 mg de KOH/g.

L'autre voie de production de dimères d'acides gras utilise des terres activées. On peut citer par exemple les brevets US-A-2 347 562, 2 426 489 et 2 793 220. Elle consiste à dimériser, à une température de 210-230 °C, les acides oléique ou linoléique en présence d'une terre activée de la famille des aluminosilicates de métaux alcalins et alcalino-terreux comme par exemple de la montmorillonite, de la sépiolite, de l'attapulgite ou de l'halloysite.

En utilisant le procédé «Emery» de la société Unilever, on obtient par exemple après réaction un produit contenant environ 55 % poids de dimères et trimères d'acides gras, le reste étant constitué principalement d'acide stéarique et isostéarique, ce dernier étant constitué d'un mélange de produits résultant de l'isomérisation squelettale de la molécule d'acide gras.

Le produit de réaction doit ensuite être purifié par distillation afin d'éliminer la fraction des acides monomères. On obtient alors un mélange dont la composition est de l'ordre de 1 % d'acides monomères, 75 % d'acides dimères, 19 % d'acides trimères et 5 % d'oligomères supérieurs.

On peut citer également la catalyse par les acides sulfoniques ainsi que par les résines échangeuses d'ions fortement acides (voir document de brevet DE-A-3 250 470 de Henkel GmbH).

On peut noter que ces différentes méthodes peuvent être utilisées à la fois sur tes acides gras et sur leurs esters correspondants (par exemple sous forme d'esters méthyliques, éthyliques, propyliques, butyliques )

La réaction d'amidification des acides gras par une amine primaire ou secondaire est connue de l'art antérieur et fonctionne entre 110 et 160 °C sans catalyseur avec élimination de l'eau au fur et à mesure qu'elle se forme, soit par utilisation d'un tiers solvant pour obtenir un azéotrope, soit par opération sans solvant, mais par élimination de l'eau par distillation sous pression réduite (voir par exemple le brevet US-A-2 089 212 et l'article de Harry Kroll et Herbert Nadeau dans J.A.O.C.S. 34, 323-326, juin 1957).

Les compositions de l'invention comprennent au moins un mélange d'alcanolamides d'huile végétale polyinsaturée polymérisée thermiquement et présentant une viscosité à 20 °C de 5 à 65 Pa.s, de préférence de 10 à 20 Pa.s. Ladite huile végétale polyinsaturée polymérisée est plus particulièrement sous la forme d'un mélange comprenant des monomères, des dimères, des trimères et des oligomères supérieurs.

Les compositions de l'invention peuvent comprendre en outre au moins des amines, des ester-amides, des ester-amines, des sels d'amines et des monoglycérides dérivant des monomères, des dimères, des trimères et des d'oligomères supérieurs d'huile végétale polyinsaturée.

L'huile végétale polyinsaturée polymérisée présente en général un indice d'acide compris entre 8 et 20. Elle dérive de préférence de l'huile de lin.

L'originalité de l'invention réside dans le fait que l'on utilise comme source de dimères une huile polyinsaturée polymérisée de viscosité à 20 °C comprise entre 5 et 65 Pa.s.

Le procédé de fabrication des compositions de l'invention comprend la réaction de transamidification d'une huile végétale polyinsaturée polymérisée thermiquement présentant une viscosité à 20 °C de 5 à 65 Pa.s avec un excès d'un aminoalcool, en présence ou non de catalyseur.

Les aminoalcools susceptibles d'être utilisés sont par exemple la monoéthanolamine, la monopropanolamine, la monoisopropanolamine, le 1-aminobutanol, le 2-amino-1-butanol, la N-méthyléthanolamine, la N-butyléthanolamine, la pentanolamine, l'hexanolamine, la cyclohexanolamine, les polyalcoolamines, ou encore les polyalcoxyglycolamines, ainsi que les polyols aminés tels que la diéthanolamine, la diisopropanolamine ou le trihydroxyméthylaminométhane. De préférence, on utilisera la diéthanolamine.

La réaction est en général conduite en l'absence de solvant et de catalyseur, sous atmosphère d'azote et à une température comprise entre 100 et 200 °C et de préférence à une température de 160 °C. Mais elle peut être également catalysée, ce qui diminue sensiblement le temps de réaction. On utilise généralement des alcoolates alcalins de type méthylate ou éthylate de lithium, sodium ou potassium. Le temps de réaction est alors de 15 à 200 minutes de préference de 80 à 120 minutes; de préférence, la réaction est arrêtée après 100 minutes.

Les huiles végétales polymérisées susceptibles d'être utilisées doivent avoir une viscosité de 5 à 65 Pa.s à 20 °C et de préférence de 10 à 20 Pa.s. Elles possèdent généralement un indice d'acide compris entre 8 et 20.

Le rapport molaire de l'aminoalcool sur l'huile polymérisée, par rapport aux moles d'acides gras contenus dans l'huile, doit être de 1 à 2 et de préférence de l'ordre de 1,5, ce qui correspond à un rapport molaire de l'aminoalcool à l'huile polymérisée de 3 à 6, de préférence voisin de 4,5.

Le produit obtenu peut être utilisé directement sans aucune purification, soit à l'état pur, soit après dilution dans un solvant adapté à l'application émulsifiante choisie. Les solvants compatibles utilisables peuvent être choisis parmi les solvants aromatiques, comme par exemple le toluène ou les xylènes, les coupes de solvants aromatiques; les monoalcools, comme par exemple le méthanol, l'éthanol, le propanol, l'isopropanol, le butanol, l'isobutanol ou le dodécanol, les diols comme par a example le monopropylène glycol et le diéthylène glycol ; tous esters de monoalcools de C1 à C4 et d'acides gras de C6 à C22, comme par exemple ceux dérivant d'huiles ou de graisses végétales ou animales, ainsi que les mêmes esters purs, tels que par exemple l'hexanoate et l'octoate de méthyle ou d'éthyle.

Les esters de monoalcools de C1 à C4 et d'acides gras de C6 à C22 dérivant d'huiles ou de graisses végétales ou animales peuvent être choisis par exemple parmi les esters méthylique et éthylique des huiles de coprah, de babassu, de palmiste, de tucum, de murumuru, de palme, de karité, d'olive, d'arachide, de kapok, de datte amère, de papayer, de coloquinte, de croton, de souchet, d'épurge, de chanvre, de hêtre, de ketmie, de pulghère, de cameline, de carthame, de niger, de tournesol, de tournesol oléique, d'hévéa, de coco, de purga, de noix, de maïs, de soja, de coton, de sorgho, de pépin de raisin, de lin, de tabac, de pin commun, d'afzellie, de chou navet, de sénevé, de moutarde brune, de bois de chine, de bancoulier, d'aleurite, d'amoora, de sapin, de cramble, de périlla, de colza érucique, de colza nouveau, de colza oléique, de sésame, de beurre de cacao, de tall oil, de germe de blé et de ricin ; et les parmi les esters méthylique et éthylique de graisses telles que le saindoux, le suif et le beurre fondu, ainsi que d'huiles de poisson, en l'état ou partiellement hydrogénées.

Dans certaines applications où il peut y avoir un contact avec l'environnement, l'usage de solvants aromatiques devra être remplacé par des solvants présentant une non-toxicité et une certaine biodégradabilité, d'où l'intérêt d'employer comme solvants des esters gras issus de bases végétales, comme par exemple de l'ester méthylique ou éthylique d'huile de colza.

Les compositions définies dans l'invention peuvent trouver un grand nombre d'applications. Elles peuvent être utilisées comme agents émulsifiants permettant, selon la nature de l'huile, de former des émulsions huile-dans-eau ou eau-dans-huile. Elles peuvent encore être utilisées comme agents dispersants de solides ou comme stabilisants de mousses dans un liquide ou dans une émulsion.

Une utilisation particulière consiste à former des dispersions stables de glace dans des milieux hydrocarbonés, par exemple dans des condensats légers de pétrole.

Les exemples suivants illustrent l'invention mais ne doivent en aucune manière être considérés comme limitatifs. Les exemples 2, 3 et 4 sont donnés à titre comparatif.

### Exemple 1

Dans un ballon d'un litre, équipé d'une agitation mécanique et chauffé par l'intermédiaire d'un bain d'huile, on introduit sous atmosphère d'azote 325 g d'huile de lin polymérisée (0,37 mole), comptée en équivalent de triglycérides et possédant une viscosité de 10 Pa.s à 20 °C avec 175 g de diéthanolamine (1,66 moles).

La composition correspondante en acides gras et oligomères d'acides gras de cette huile de lin polymérisée est la suivante :
- acides gras monomères = 48,8 %,
- acides gras dimères = 31,4 %,
- acides gras trimères = 13,0 % et
- oligomères supérieurs = 5,8 %.

L'indice d'acide du produit est égal à 10.

On chauffe le mélange jusqu'à 160 °C. Après 15 à 20 minutes de réaction à 160 °C, le produit devient homogène et limpide. On maintient cette température de 160 °C pendant 100 minutes avant de refroidir le mélange.

La dilution du produit s'effectue dans le ballon de réaction en introduisant 500 g d'une coupe d'aromatiques dont l'intervalle de distillation est compris entre 180 et 215 °C.

Le mélange ainsi obtenu est un liquide jaune, d'une densité à 25 °C de 950 kg/m³, d'une viscosité à 20 °C de 0,212 Pa.s, d'un point éclair > 60 °C et d'un point d'écoulement < 20 °C.

Le temps total de cette synthèse en tenant compte des temps de chauffage est compris entre 2 et 3 heures.

### Exemple 2 (comparatif)

Dans le même appareillage que pour l'exemple 1, on introduit 330 g d'un mélange d'acides gras issus d'une huile de tournesol, dont la composition est la suivante :
- acide palmitique = 6 %,
- acide stéarique = 5 %,
- acide oléique = 18 %,
- acide linoléique = 69 %,
et 35 cm³ d'une résine échangeuse d'ions fortement acide de type sulfonique, préalablement séchée (résine Amberlyst 15®).

On chauffe progressivement sous atmosphère d'azote ce mélange jusqu'à 130 °C en maintenant une agitation.

Après 7 à 8 heures de réaction à 130 °C, la conversion en oligomères d'acides gras est de 54,5 % (selon l'analyse obtenue par GPC).

Après filtration de la résine échangeuse d'ions, on récupère 295 g d'un mélange d'acides gras mono-, di et trimères, dont l'indice d'acide est égal à 195. Ceci correspond à 1,026 moles d'acidité grasse. Sur ce mélange on introduit 1,54 moles de diéthanolamine, soit 161,6 g, et l'on chauffe progressivement jusqu'à une température de 160 °C. Dès que l'on atteint la température de 130°C, on commence la mise sous vide de l'appareillage de façon à faciliter l'élimination de l'eau de réaction jusqu'a obtenir, en fin de réaction, un vide de l'ordre de 50 à 60 mm de mercure. Après 7 heures de réaction, on à récupéré 29 g d'eau contenant 2,1 g de diéthanolamine.

La composition du mélange réactionnel est déterminé par dosage. On trouve 1,18 moles d'amine totale, 0,8 mole de diéthanolamine libre, 0,38 mole d'amino-esters, environ 0,02 mole de sels d'amine et de l'ordre de 2,2 moles d'amides, par kilogramme du produit fabriqué.

L'acidité résiduelle du produit correspond à un indice d'acide de l'ordre de 1. La dilution du produit de réaction est réalisée en ajoutant 422 g du solvant utilisé dans l'exemple 1, consistant en une coupe de solvants aromatiques.

Après un temps de stockage de 72 heures, on remarque l'apparition, dans le produit dilué, de petits cristaux blancs. L'analyse de ce solide, par le couplage chromatographie/spectrométrie de masse, montre qu'il s'agit de la N,N'-bis (2-hydroxyéthyl) pipérazine. Ce produit résulte de la déshydratation de deux molécules de diéthanolamine. Un dosage par chromatographie gazeuse permet d'estimer à 3 % poids la quantité de ce sous produit dans le produit non dilué.

Le temps total de cette synthèse en incluant les temps de chauffage et l'étape de filtration est de l'ordre de 18 heures.

### Exemple 3 (comparatif)

On introduit dans un réacteur de 0,5 litre en acier inoxydable et tenant à la pression 350 g d'acide oléique commercial (dont la teneur en acide oléique est d'environ 65 % et dont la somme des acides gras insaturés à 18 atomes de carbone est de l'ordre de 80 %) et 35 g de terre activée du type montmorillonite (Tonsil Optimum FF®).

On chauffe avec agitation ce mélange sous atmosphère d'azote et montée de la température pour atteindre 230 °C. Après 5 heures à 230 °C, l'analyse GPC montre que l'on a fabriqué environ 55 % d'un mélange dimères et trimères d'acides gras et 45 % d'acides monomères contenant 25 % d'acides palmitique et stéarique et environ 20 % d'un mélange d'isomères d'acides gras branchés plus ou moins saturés.

Après filtration de la terre activée sur un filtre papier, on récupère 308 g de produit qui va être amidifié par de la diéthanolamine, selon la même procédure que pour l'exemple 2.

On ajoute 170,8 g de diéthanolamine (1,63 moles) et après 6 heures de réaction, on récupère 30 g d'eau contenant 2,3 g de diéthanolamine. L'indice d'acide résiduel est égal à 1,4 mg de KOH/g de produit et la teneur en N,N'-bis (2-hydroxyéthyl) pipérazine obtenue par analyse chromatographique est de 2,8 %. On récupère *in fine* 448 g de produit utilisable directement en l'état, ou après une dilution visant à en diminuer sensiblement la viscosité afin qu'il puisse être manipulé facilement.

Le temps de réaction nécessaire pour obtenir le produit a été d'environ 15 heures.

### Exemple 4 (comparatif)

On introduit 345 g d'esters méthyliques d'huile de tournesol de composition identique au mélange d'acides gras utilisé dans l'exemple 2 et 35 cm³ de résine échangeuse d'ions fortement acide (Amberlyst 15®) et on chauffe progressivement jusqu'à 130 °C sous atmosphère d'azote. Après 6 heures de réaction, on obtient une conversion en oligomères (dimères + trimères) de 56 % selon l'analyse GPC.

Après filtration de la résine on récupère 310 g de produit sur lequel on ajoute 162 g de diéthanolamine.

A noter que l'amidification d'un ester, contrairement à celle d'un acide, demande à être catalysée. La catalyse par un alcoolate alcalin est le plus souvent pratiquée.

On chauffe donc le mélange et dès que l'on atteint 100-110 °C, on ajoute 0,023 mole de méthylate de sodium, soit 4,16 g d'une solution à 30% dans le méthanol. On recueille le méthanol qui part de la réaction de transamidification et on maintient le chauffage jusqu'à 130°C pendant environ 2 heures sous pression réduite pour faciliter le départ du méthanol.

Le produit ainsi fabriqué est dilué à 50 % poids avec la coupe de solvants aromatiques utilisée dans l'exemple 1.

### Exemple 5

Dans un réacteur agité en acier inoxydable, d'une capacité de 120 litres, on introduit 65 kg d'huile de lin polymérisée d'une viscosité de 10 Pa.s à 20 °C et 35 kg de diéthanolamine.

On chauffe le mélange jusqu'à 160°C sous atmosphère d'azote. La température de consigne est atteinte après 80 minutes et on maintient cette température de 160 °C pendant environ 100 minutes.

On dilue le produit obtenu après refroidissement vers 120 °C dans une cuve agitée contenant 100 kg d'ester méthylique d'huile de ricin pour obtenir une dilution pondérale de 50 %.

La composition du produit fabriqué avant dilution, déterminée par dosage, est la suivante :
- 1,39 moles/kg d'amine totale,
- 1,3 moles/kg de diéthanolamine libre,
- 0,09 mole/kg d'amino-esters,
- 0,6 mole/kg de glycérine libre,
- 0,12 mole/kg de monoglycérides,
- de l'ordre de 0,17 mole/kg de sels d'acide gras de diéthanolamine et
- 1,8 moles/kg d'amides d'acides gras.

La viscosité à 20 °C du produit avant dilution est de 12,710 Pa.s et après dilution à 50/50 en poids dans de l'ester méthylique de ricin, la viscosité à 20 °C est de 0,567 Pa.s.

### Exemple 6

On dissout à l'aide d'un barreau magnétique 0,15 g de solution d'émulsifiant fabriqué dans l'exemple 1 (produit dilué à 50 % avec une coupe d'aromatiques), avec 90 g d'un mélange d'hydrocarbures représentant un condensat de pétrole.

La composition pondérale du condensat est la suivante :
- pour les molécules ayant moins de 11 atomes de carbone :
   - 20 % de paraffines et d'isoparaffines, 48 % de naphtènes, 10 % d'aromatiques ; et
- pour les molécules ayant au moins 11 atomes de carbone :
   - 22 % d'un mélange de paraffines, d'isoparaffines, de naphtènes et d'aromatiques.

Après 10 à 15 minutes d'agitation continue, on ajoute 10 g d'eau à ce mélange et on agite à l'aide d'un agitateur de type turbine, à 8000tr/min pendant 30 secondes. L'émulsion formée est fine et stable. Elle est de type eau-dans-huile.

### Exemple 7

On dissout 0,3 g de solution d'émulsifiant fabriqué dans l'exemple 5 (produit dilué à 50 % en poids avec de l'ester méthylique de ricin), dans 180 g d'eau, puis on ajoute 20 g d'huile de colza raffinée et on agite à 8000 tr/min pendant 30 secondes à l'aide d'un agitateur de type turbine. L'émulsion formée est fine et stable. Elle est de type huile-dans-eau.

Les exemples qui précèdent peuvent être répétés avec des résultats analogues en substituant les réactifs et/ou les conditions générales ou particulières décrites dans l'invention à ceux mis en oeuvre dans ces exemples.

## Revendications

1. Composition **caractérisée en ce qu'**elle comprend au moins un mélange d'alcanolamides d'huile végétale polyinsaturée polymérisée thermiquement et présentant une viscosité à 20 °C de 5 à 65 Pa.s.

2. Composition selon la revendication 1, **caractérisée en ce qu'**elle comprend en outre au moins des amines, des ester-amides, des ester-amines, des sels d'amines et des monoglycérides.

3. Composition selon la revendication 1 ou 2, **caractérisée en ce que** ladite huile végétale polyinsaturée polymérisée est sous la forme d'un mélange comprenant des monomères, des dimères, des trimères et des oligomères supérieurs.

4. Composition selon l'une des revendications 1 à 3, **caractérisée en ce que** l'huile végétale polyinsaturée polymérisée présente une viscosité à 20 °C de 10 à 20 Pa.s.

5. Composition selon l'une des revendications 1 à 4, **caractérisée en ce que** l'huile végétale polyinsaturée polymérisée présente un indice d'acide compris entre 8 et 20.

6. Composition selon l'une des revendications 1 à 5, **caractérisée en ce que** l'huile végétale polyinsaturée polymérisée dérive de l'huile de lin.

7. Composition selon l'une des revendications 1 à 6, **caractérisée en ce qu'**elle comprend en outre au moins un solvant.

8. Composition selon la revendication 7, **caractérisée en ce que** ledit solvant est choisi parmi les solvants aromatiques, les coupes de solvants aromatiques; les monoalcools, les diols, tous esters de monoalcools de C1 à C4 et d'acides gras de C6 à C22, dérivant d'huiles ou de graisses végétales ou animales, ainsi que les mêmes esters purs.

9. Procédé de fabrication d'une composition selon l'une des revendications 1 à 8, **caractérisé en ce qu'**il comprend la réaction de transamidification d'une huile végétale polyinsaturée polymérisée thermiquement présentant une viscosité à 20 °C de 5 à 65 Pa.s avec un excès d'un aminoalcool, en présence ou non de catalyseur.

10. Procédé selon la revendication 9, **caractérisé en ce que** l'huile végétale polyinsaturée polymérisée présente une viscosité à 20 °C de 10 à 20 Pa.s.

11. Procédé selon la revendication 9 ou 10, **caractérisé en ce que** l'huile végétale polyinsaturée polymérisée présente un indice d'acide compris entre 8 et 20.

12. Procédé selon la revendication 9 à 11, **caractérisé en ce que** l'on transamidifie de l'huile de lin polymérisée thermiquement.

13. Procédé selon l'une des revendications 9 à 12, **caractérisé en ce que** le rapport molaire de l'aminoalcool à l'huile polymérisée est de 3 à 6.

14. Procédé selon la revendication 13, **caractérisé en ce que** le rapport molaire de l'aminoalcool à l'huile polymérisée est voisin de 4,5.

15. Procédé selon l'une des revendications 9 à 14, **caractérisé en ce que** l'aminoalcool utilisé est la diéthanolamine.

16. Procédé selon l'une des revendications de 9 à 15, **caractérisé en ce que** la transamidification est obtenue avec ou sans catalyseur, après un temps de chauffage de 15 à 200 minutes.

17. Procédé selon la revendication 16, **caractérisé en ce que** la transamidification est obtenue après un temps de chauffage de 80 à 120 minutes.

18. Procédé selon l'une des revendications de 9 à 17, **caractérisé en ce que** la température de réaction est de 100 à 200 °C.

19. Procédé selon la revendication 18, **caractérisé en ce que** la température de réaction est de l'ordre de 160 °C.

20. Utilisation d'une composition selon l'une des revendications 1 à 8 comme agent émulsifiant permettant, selon la nature de l'huile, de former une émulsion huile-dans-eau ou eau-dans-huile.

21. Utilisation d'une composition selon l'une des revendications 1 à 8 comme agent dispersant de solides ou comme stabilisant de mousses dans un liquide ou une émulsion.

22. Utilisation selon la revendication 21 pour former une dispersion stable de glace dans un condensat de pétrole léger.

## Claims

1. A composition **characterized in that** it comprises at least a mixture of alcanolamides of a thermally polymerized polyunsaturated vegetable oil having a viscosity of 5 to 65 Pa.s at 20°C.

2. A composition according to claim 1 **characterized in that** it further comprises at least amines, ester-amides, ester-amines, amine salts and monoglycerides.

3. A composition according to claim 1 or 2, **characterized in that** said polymerized polyunsaturated vegetable oil also is in the form of a mixture comprising monomers, dimers, trimers and higher oligomers.

4. A composition according to any one of claims 1 to 3, **characterized in that** the polymerized polyunsaturated vegetable oil has a viscosity at 20°C of 10 to 20 Pa.s.

5. A composition according to any one of claims 1 to 4, **characterized in that** the polymerized polyunsaturated vegetable oil has an acid index of between 8 and 20.

6. A composition according to any one of claims 1 to 5, **characterized in that** the polymerized polyunsaturated vegetable oil is derived from linseed oil.

7. A composition according to any one of claims 1 to 6 **characterized in that** it also comprises at least one solvent.

8. A composition according to claim 7, **characterized in that** said solvent is selected from among aromatic solvents, aromatic solvent fractions, monoalcohols, diols, all esters of C1 to C4 monoalcohols and C6 to C22 fatty acids derived from vegetable or animal oils or fats, as well as the same esters pure.

9. A process for production of a composition according to one of claims 1 to 8, **characterized in that** it comprises the transamidification reaction of a thermally polymerized polyunsaturated vegetable oil having a viscosity at 20°C of 5 to 65 Pa.s with an excess of amino alcohol, in the presence or absence of a catalyst.

10. A process according to claim 9, **characterized in that** the polymerized vegetable oil is obtained by polymerization of a vegetable oil has a viscosity at 20°C of 10 to 20 Pa.s.

11. A process according to claims 9 to 10, **characterized in that** the polymerized polyunsaturated vegetable oil has an acid index of between 8 and 20.

12. A process according to claim 9 or 11, **characterized in that** thermally polymerized linseed oil is being transamidified.

13. A process according to claims 9 to 12, **characterized in that** the molar ratio of the amino alcohol to the polymerized oil is 3 to 6.

14. A process according to claim 13, **characterized in that** the molar ratio of the amino alcohol to the polymerized oil is close to 4.5.

15. A process according to one of claims 9 to 14, **characterized in that** the amino alcohol used is diethanolamine.

16. A process according to one of claims 9 to 15, **characterized in that** the transamidification is obtained, with or without catalyst after a heating time of 15 to 200 minutes.

17. A process according to claim 16, **characterized in that** the transamidification is obtained after a heating time of 80 to 120 minutes.

18. A process according to one of claims 9 to 17, **characterized in that** the reaction temperature is 100 to 200°C.

19. A process according to claim 18, **characterized in that** the reaction temperature is on the order of 160°C.

20. The use of a composition according to one of claims 1 to 8 as an emulsifying agent which makes it possible, depending on the nature of the oil, to form an oil-in-water or water-in-oil emulsion.

21. The use of a composition according to one of claims 1 to 8 as a solid-dispersing agent or as a stabilizer for foams in a liquid or an emulsion.

22. The use according to claim 21 for forming a stable ice dispersion in a light petroleum condensate.

## Patentansprüche

1. Zusammensetzung, **dadurch gekennzeichnet, dass** sie wenigstens ein Gemisch von Alkanolamiden eines polyungesättigten Pflanzenöls umfasst, das thermisch polymerisiert ist und eine Viskosität bei 20°C von 5 bis 65 Pa.s aufweist.

2. Zusammensetzung nach Anspruch 1, **dadurch gekennzeichnet, dass** sie im übrigen wenigstens Amine, Esteramide, Esteramine, Salze von Aminen und Monoglyceride umfasst.

3. Zusammensetzung nach Anspruch 1 oder 2, **dadurch gekennzeichnet, dass** das polyungesättigte polymerisierte Pflanzenöl in Form einer Mischung vorliegt, die Monomere, Dimere, Trimere und höhere Oligomere umfasst.

4. Zusammensetzung nach einem der Ansprüche 1 bis 3, **dadurch gekennzeichnet, dass** das polyungesättigte polymerisierte Pflanzenöl eine Viskosität bei 20°C von 10 bis 20 Pa.s aufeist.

5. Zusammensetzung nach einem der Ansprüche 1 bis 4, **dadurch gekennzeichnet, dass** das polyungesättigte polymerisierte Pflanzenöl einen Säureindex zwischen 8 und 20 aufweist.

6. Zusammensetzung nach einem der Ansprüche 1 bis 5, **dadurch gekennzeichnet, dass** das polyungesättigte polymerisierte Pflanzenöl von Leinöl abgewandelt ist.

7. Zusammensetzung nach einem der Ansprüche 1 bis 6, **dadurch gekennzeichnet, dass** sie im übrigen wenigstens ein Lösungsmittel umfasst.

8. Zusammensetzung nach Anspruch 7, **dadurch gekennzeichnet, dass** das Lösungsmittel unter den aromatischen Lösungsmitteln, den aromatischen Lösungsmittelschnitten, den Monoalkoholen, den Diolen, alle Estern von C1-bis C4-Monoalkoholen und C6- bis C22-Fettsäuren, die sich von pflanzlichen oder tierischen Ölen oder Fetten ableiten, sowie den gleichen reinen Estern gewählt ist.

9. Herstellungsverfahren einer Zusammensetzung nach einem der Ansprüche 1 bis 8, **dadurch gekennzeichnet, dass** es die Transamidifizierungsreaktion eines polyungesättigten thermisch polymerisierten Pflanzenöls mit einer Viskosität bei 20°C von 5 bis 65 Pa.s mit einem Überschuss eines Aminoalkohols gegebenenfalls in Gegenwart eines Katalysators umfasst.

10. Verfahren nach Anspruch 9, **dadurch gekennzeichnet, dass** das polyungesättigte polymerisierte Pflanzenöl eine Viskosität bei 20°C von 10 bis 20 Pa.s aufweist.

11. Verfahren nach einem der Ansprüche 9 oder 10, **dadurch gekennzeichnet, dass** das polyungesättigte polymerisierte Pflanzenöl einen Säureindex zwischen 8 und 20 aufweist.

12. Verfahren nach einem der Ansprüche 9 bis 11, **dadurch gekennzeichnet, dass** man das thermisch polymerisierte Flachsöl transamidifiziert.

13. Verfahren nach einem der Ansprüche 9 bis 12, **dadurch gekennzeichnet, dass** das molare Verhältnis von Aminoalkohol zu polymerisiertem Öl 3 bis 6 ist.

14. Verfahren nach Anspruch 13, **dadurch gekennzeichnet, dass** das molare Verhältnis von Aminoalkohol zu polymerisiertem Öl nahe 4,5 ist.

15. Verfahren nach einem der Ansprüche 9 bis 14, **dadurch gekennzeichnet, dass** der verwendete Aminoalkohol ein Diethanolamin ist.

16. Verfahren nach einem der Ansprüche 9 bis 15, **dadurch gekennzeichnet, dass** die Transamidifizierung mit oder ohne Katalysator nach einer Aufheizzeit von 15 bis 200 Minuten erhalten wird.

17. Verfahren nach Anspruch 16, **dadurch gekennzeichnet, dass** die Transamidifizierung nach einer Heizzeit von 80 bis 120 Minuten erhalten wird.

18. Verfahren nach einem der Ansprüche 9 bis 17, **dadurch gekennzeichnet, dass** die Reaktionstemperatur 100 bis 200°C ist.

19. Verfahren nach Anspruch 18, **dadurch gekennzeichnet, dass** die Reaktionstemperatur in der Größenordnung von 160°C ist.

20. Verwendung einer Zusammensetzung nach einem der Ansprüche 1 bis 8 als emulgierendes Reagenz, das es ermöglicht, gemäß der Natur des Öls eine Emulsion Öl in Wasser oder Wasser in Öl zu bilden.

21. Verwendung einer Zusammensetzung nach einem der Ansprüche 1 bis 8 als dispergierendes Reagenz von Feststoffen oder als Schaumstabilisierungsmittel in einer Flüssigkeit oder einer Emulsion.

22. Verwendung nach Anspruch 21, um eine glanzstabile Dispersion in einem Kondensat eines leichten Erdöls zu bilden.
